Europäisches Patentamt

European Patent Office

Office européen des brevets

(19)

(11) Publication number: **0 298 669**
**B1**

# EUROPEAN PATENT SPECIFICATION

(12)

(45) Date of publication of the patent specification: 05.12.90

(51) Int. Cl.⁵: **B01J 19/00, C12Q 1/68**

(21) Application number: **88306007.1**

(22) Date of filing: **01.07.88**

(54) **Performing nucleic acid reactions.**

(30) Priority: **10.07.87 GB 8716279**

(43) Date of publication of application:
**11.01.89 Bulletin 89/2**

(45) Publication of the grant of the patent:
**05.12.90 Bulletin 90/49**

(84) Designated Contracting States:
**AT BE CH DE FR GB IT LI LU NL SE**

(56) References cited:
**EP-A- 0 187 699**
**EP-A- 0 198 413**
**EP-A- 0 223 618**
**WO-A-84/03444**
**WO-A-84/03715**
**US-A- 4 517 160**

(73) Proprietor: **AMERSHAM INTERNATIONAL plc,
Amersham Place, Little Chalfont Buckinghamshire
HP7 9NA(GB)**

(72) Inventor: **Ortlepp, Stephen Andrew, 94 Hermitage Road
St. Johns, Woking Surrey, GU21 ITQ(GB)**
Inventor: **McKay, Ian Alexander, 17 Sarre Road, London,
NW2 3SN(GB)**

(74) Representative: **Pennant, Pyers et al, Stevens, Hewlett &
Perkins 5 Quality Court Chancery Lane, London,
WC2A 1HZ(GB)**

**Description**

This invention relates to a method for performing nucleic acid reactions and for manipulating nucleic acids (by performing two or more such reactions in sequence). Present methods involve many manipulations in small test tubes – usually microfuge tubes of approximately 1.8 ml capacity, and suffer in particular from two shortcomings:

a) Enzymes, buffers, nucleotides and other reagents have to be transferred from stock tubes kept at -20°C to tubes containing the nucleic acid sequences.

b) Present methods require the researcher to handle large numbers of tubes, pre-mix several solutions and repeatedly pipet very small volumes, which makes such operations, tedious, lengthy, prone to error and difficult to automate.

This invention is based on the idea of simplifying such manipulations by pre-dispensing and drying some or all of the reagents involved. Certain nucleic acid enzymes are commercially available in bulk as the freeze dried solid, but have not, so far as is known, been supplied in pre-dispensed form.

EP-A 198 413 describes a machine for performing assays, in which capture and conjugate reagents may be provided in freeze-dried form.

WO-A 8 403 715 describes a nucleic acid assay method and kit, in which enzymes and other reagents may be present separately in lyophilized form.

WO-A 8 403 444 describes an antibody capture plate assay, in which antibodies may be provided in lyophilized form.

EP-A 187 699 and EP-A 223 618 describe machines for automated DNA sequencing operations, in which reagents are dispensed in fluid form.

In one aspect, the invention provides a method of performing a nucleic acid reaction, by providing a reaction vessel containing a predetermined amount of a reagent for the reaction, comprising a mixture of an enzyme capable of acting on nucleic acids and a buffer and including components of the reaction except a nucleic acid substrate, in a dried state, adding to the vessel aqueous fluid containing a nucleic acid sequence, and incubating the vessel.

In another aspect, the invention provides a method of performing a nucleic acid manipulation, by providing a first and at least one subsequent vessel, each vessel containing a predetermined amount of a reagent for a nucleic acid reaction in a dried state, adding to the first vessel aqueous fluid containing a nucleic acid sequence, and transferring a fluid reaction product from the first vessel to the or each subsequent vessel.

The nature of the nucleic acid sequence is not important. It may for example be DNA or RNA or a natural or synthetic oligomer.

The reagent for a nucleic acid reaction is preferably an enzyme or a substrate for such an enzyme, which may optionally be labelled with a radioactive or other marker. Examples of enzymes include restriction enzymes, nucleic acid polymerases and nucleic acid modification enzymes. Examples of substrates include nucleotides and derivatives thereof, and nucleic acid chains. Generally, the reagent is pre-dispensed in liquid form and dried in the reaction vessel. Drying conditions may be at elevated or ambient temperature, or at lower temperature as by freeze-drying or lyophilisation. Some reagents may require stabilizers while drying down. It is generally convenient to dry down a buffered solution of the reagent, thus giving rise to a dried mixture of reagent and buffer. When the reagent is an enzyme, the dried mixture preferably includes all components of the reaction (except a nucleic acid substrate) including for example a cofactor, and a substrate for the enzyme where two or more substrates are required for reaction.

In the context of enzymes, the term "buffer" is used to include all the other components, not merely those concerned with pH control, used to maintain the enzyme. In practice, two different buffers are used, a storage (or dilution or reconstitution) buffer to preserve the enzyme from loss of essential function during storage, and a reaction (or assay or activity) buffer to maintain conditions for the enzyme to perform its essential function. The overall composition of these two buffers are different, although individual components may overlap. The storage buffer typically contains glycerol or protein such as albumin. The reaction buffer typically contains salts, e.g. sodium, magnesium or potassium chloride or sulphate, and nucleotides or polynucleotides, which act as co-factors. The reaction buffer would not contain glycerol. The pH conditions maintained by the two buffers are often different.

There follow details of the storage and reaction buffers for three commercially available enzymes to illustrate these differences:

<u>Bam H1</u> restriction endonuclease supplied by GIBCO BRL in freeze-dried form

| Storage | Reaction |
| --- | --- |
| 50mM Tris-HCl (pH 7.5) | 20mM Tris-HCl (pH 8.0) |
| 0.5mM Na$_2$EDTA | 100mM NaCl |
| 1mM DTT | 7mM MgCl$_2$ |
| 500 µg/ml BSA | 2mM 2-mercaptoethanol |
| 50% (V/v) glycerol | |

<u>Reverse transcriptase</u> RAV-2 from Amersham International plc.

| Storage | Reaction |
| --- | --- |
| 200mM phospate (K$^+$) (pH 7.2) | 50mM Tris-HCl (pH 8.0) |
| 2mM DTT | 10mM MgCl$_2$ |
| 0.2% Nonidet P-40 (TM) | 50mM KCl |
| 50 % glycerol | 3mM DTT |
| | 20 µg/ml Poly A, oligo dT |
| | 0.1% Nonidet P-40 |

<u>E. coli DNA polymerase 1 "Klenow fragment"</u> from Amersham International plc

| Storage | Reaction |
| --- | --- |
| 50mM phosphate (K$^+$) (pH 6.5) | 67 mM phosphate (K$^+$) (pH 7.4) |
| 10mM 2-mercaptoethanol | 6.7mM MgCl$_2$ |
| 50% glycerol | 1mM 2-mercaptoethanol |
| | 33µM dNTP |

According to this invention, the reagent preferably comprises a dried down mixture of an enzyme and its reaction (not its storage) buffer. The arrangement has the advantage that no preparation is required of the aqueous fluid containing the nucleic acid sequence required to complete the ingredients needed for the nucleic acid reaction. For example, a solution of the nucleic acid sequence in water or in any convenient aqueous fluid could be used. While it would be perfectly possible (though not preferred) to include a simple reaction component such as NaCl with the nucleic acid sequence, the inclusion of a complex or macromolecular reaction component would tend to negate the value of the invention.

It is suprising that the process permits extended stable storage of reaction mixtures, unlike enzymes stored in aqueous reaction buffer.

It has been found that:-

- Enzymes for nucleic acid manipulation can be dried without loss of essential function;
- Substrates for those enzymes can be dried without loss of essential function;
- Reconstitution of enzymes and substrates with an aqueous solution of nucleic acid sequence will allow rapid manipulation of the nucleic acid sequence;
- Dried substrates and enzymes can be stored for periods in excess of six months at -20°C without loss of essential function;
- Dried substrates and enzymes can be transported without loss of essential function.

Disposable reaction vessels are available commercially. For example, Dynatech supplies "Removawells" in strips of 12, which are suitable for nucleic acid manipulations. According to a preferred feature of this invention, predetermined amounts of the reagents for nucleic acid reactions are pre-dispensed into disposable reaction vessels. The advantages of such a system include:-

- Wells containing lyophilised enzymes or substrates can be easily stored at convenient temperatures; e.g. room temperature, 4°C or -20°C.
- Wells are used once and then discarded, with no risk of contamination;
- Wells can be used in labelled racks, thus avoiding the need to label individual vessels;
- Reduced manipulation, by avoiding the need to aliquot enzymes, substrates or buffers;
- All apparatus and support equipment can be supplied in one format convenient for use;
- Well adapted to automation.
- Better reproducibility from experiment to experiment.

EP 0 298 669 B1

The nature of nucleic acid reaction or manipulation envisaged is not critical to the invention. The following experimental section gives details of four such manipulations: restriction; nuclease digestion; DNA modification; cDNA synthesis; sequencing. But the invention is equally suitable for other nucleic acid reactions and manipulations, including sequencing by use of non-radioactive labels; ligation; and SI nuclease digestion.

The wells used in the Examples were Dynatech Immunolon II "Removawells".

Example 1

Restriction Endonuclease Digestion

All enzymes were dried down at room temperature in 60µl of 1x core buffer: 50mM Tris pH 8.0, 50mM NaCl, 10mM MgCl$_2$ using a high capacity vacuum pump connected to a desiccator. Once dried, enzymes have been left at -20°C in a sealed plastic container for 3 days before use. 40 units of enzyme retained sufficient activity to digest 1ug of pBR322 at 37°C in 2 hours.

The following restriction enzymes have been dried down in this manner. All retain activity when reconstituted with solutions containing DNA.

EcoRI BamHI HindIII PstI SalI PvuII HincII BglI EcoRV

Example 2

DNA Modifying Ezymes

Three enzymes, polynucleotide kinase (PNK) (4U), T4 DNA ligase (2.5U) and DNA polI (Klenow) (3U) were dried down in 30µl TE buffer (10mM Tris-HCl, 1mM EDTA pH 8.0). In the cases of PNK and ligase, the solutions contained ATP at 1mM concentration. The enzymes were resuspended by adding DNA resuspended in 30ul of 1x buffer (67mM Tris-HCl (pH7.4), 10mM MgCl$_2$, 1mM ATP) and reactions were carried out as normal. 5' endlabeiling (PNK) and filling-in (Klenow) were monitored by incorporation of a radioactive label followed by autoradiography. Both enzymes were found to work efficiently. Ligation efficiency was judged by gel electrophoresis of the ligation mixtures and was found to be as efficient as control ligations carried out under standard conditions.

Example 3

cDNA Synthesis

The components were
Oligo-dT
Human placental ribonuclease inhibitor
Reverse transcriptase (avian myeloblastoma virus)
4 dNTP's (including a trace amount of radioactively labelled dCTP)
Reverse transcriptase buffer (reaction buffer, see above).

All these first strand components sufficient to transcribe 1µg of mRNA were dried down together. They were resuspended by adding 1µg globin RNA in 50µl water and the first strand reaction was allowed to proceed as recommended in a cDNA synthesis manual (Amersham International RPN 1256). Total incorporation of $^{32}$P was measured. The reaction was shown to work.

Example 4

Dideoxy Sequencing

1. Preparation of wells

The sequencing solutions were prepared and premixed as follows:
A reaction: A 0.01M stock solution of dATP was diluted to 0.0005M.
Wells were labelled P, K, A, C, G, T, CHA, CHC, CHG, CHT.
To these were added the following:
P: 15-mer sequencing primer (2ng)
10 x Klenow buffer (0.05M Tris-HCl, pH 7.5, 0.05M NaCl, 0.01M MgCl$_2$) 1.5µl
K: 1 unit DNA polymerase I (Klenow fragment).
A: 1.0µl of solution A°; 1.0µl of solution ddA (2'3' dideoxyadenosine triphosphate).
C: 1.0µl of solution C°; 1.0µl of solution ddC.
G: 1.0µl of solution G°; 1.0µl of solution ddG.
T: 1.0µl of solution T°; 1.0µl of solution ddT.
CHA: 1.0µl of 0.0005M mixture of all four dNTPs.

4

CHC: 1.0µl of 0.0005M mixture of all four dNTPs.
CHG: 1.0µl of 0.0005M mixture of all four dNTPs.
CHT: 1.0µl of 0.0005M mixture of all four dNTPs.

|  | A° | C° | G° | T° |
|---|---|---|---|---|
| 0.5mM dATP | 1µl | 20µl | 20µl | 20µl |
| 0.5mM dCTP | 20µl | 1µl | 20µl | 20µl |
| 0.5mM dGTP | 20µl | 20µl | 1µl | 20µl |
| 0.5mM dTTP | 20µl | 20µl | 20µl | 1µl |
| 1 × TE buffer | 20µl | 20µl | 20µl | 20µl |

The wells were dried down, sealed with clear tape and stored at -20°C.

### 2. The Sequencing Reactions

1. 13µl of the DNA to be sequenced (in water or TE buffer) was added to well P. The cover seal was replaced and the well was placed at 90°C for 5 minutes. The template/primer mix was then allowed to cool down to room temperature (approx. 5 minutes).

2. The mixture was transferred to well K.

3. 2µl radioactive label ([α$^{32}$P]dATP or [α$^{35}$S]dATP S)was added to the template/primer/enzyme (tpe) mixture.

4. 3.5µl of the tpe mix was added to each of the four wells A, C, G, T. The cover seals were replaced.

5. The wells were incubated in an incubator for 15 ($^{32}$P) or 20 ($^{35}$S) minutes.

6. The contents of each of the four wells labelled A, C, G, T were transferred to four wells labelled CHA, CHC, CHG, CHT. The cover seals were replaced.

7. Incubations were continued for 15 or 20 minutes, again depending on the radiolabel.

8. 3µl of loading buffer were added.

9. The wells were placed at 90°C for 3 minutes and the fluid reaction products were loaded on a sequencing gel.

### Results

1. Autoradiograph of $^{32}$P labelled DNA after electrophoresis. The result showed:
1. Good resolution of DNA.
2. Sequencing ladder can be read easily.
3. Little background and no compression or "stuttering" of enzyme.
2. Essentially similar results were obtained when using a $^{35}$S radiolabel.

### Example 4b

### Dideoxy Sequencing

This example is identical to Example 4, except for the following modifications.

1. The radiolabel was predispensed and dried in a well.
2. Additional transfer involved.

A. Preparation of wells.

1µl of [α -$^{32}$P] dATP and [α $^{35}$SdATP α-S] was dispensed into a well marked RL, dried and stored at -20°C. The wells were thus labelled.
P K RL A C G T CHA, CHC CHG CHT

B. Sequencing reaction

Steps 3 and 4 of Example 4 were replaced by the following:

3. The mixture was transferred from well K into well RL.
4. 3.5µl of the mix in RL was transferred to each of the four wells A, C, G, T. Cover seals were replaced.

Proceed as from step 5 in Example 4.

## Results

Results showed as in Example 4.

## Example 4c

### Dideoxy sequencing

This is indentical to Example 4 and Example 4b except with the following modifications.
The wells once dried were stored at 4°C, 20°C, 37°C.

### Example Results

The results showed:

1. Good resolution of DNA
2. Sequencing ladder can be read easily.
3. For wells stored for 1 month or more; results were as in 1 and 2 for the wells stored at 4°C, 20°C - deterioration of result in the case of wells stored at 37°C.

## Example 4d

Dideoxy sequencing with heat stable DNA Polymerase from Thermus Aquaticus (Taq Polymerase).
The conditions were identical except for the following:

1. Preparation of wells. Well 'K' replaced by well TP.
TP: 3 units of Taq Polymerase.
2. Sequencing reactions. As in Example 4, except:
Step 5 becomes:
5. The wells were incubated in an incubator at 55°C for 15 ($^{32}$P) and 20 ($^{35}$S) minutes.
Step 7 becomes:
7. Incubations were continued at 55°C for 15 or 20 minutes.

## Results

Results showed:

1. Good resolution of DNA.
2. Sequencing ladder can easily be read to greater than 80 nucleotides.

## Example 4e

### Dideoxy sequencing

This is identical to Example 4d except with the following modifications.

Sequencing reactions. Step 1 becomes:
1. 13µl DNA to be sequenced (in water or TE buffer) was added to well P.
Step 3 becomes:
3. 2µl radioactive label ([α $^{32}$P]dATP or [α $^{35}$S] SdATP αS) was added to the mixture. The cover seal was replaced and the well placed at 65°C for 3 minutes. The well was then allowed to cool to room temperature.
Continue as step 4 of Example 4d.

## Results

Results showed as in Example 4d.

## Example 5

### Dideoxy sequencing with modified T7 polymerase (Sequenase $^{TM}$).

Sequencing solutions and reaction conditions were as recommended by manufacturer.

The sequencing solutions were prepared and premixed as follows:

e.g.

Labelling mix: a 5 x labelling mix

7.5µM GTP

7.5µM dCTP

7.5µM dTTP

was diluted to 1 x concentration.

e.g.

Termination mixes: ddATP (tA) mix:

80µM dGTP, 80µM dATP

80µM dCTP, 80µM dTTP

8µM ddATP, 50µM NaCl

Wells were labelled P T7 LM tA tC tG tT

P: 1µl M13 universal primer (l7mer) (3.2ng)

2µl Buffer (5x)

200mM Tris HCl pH7.5

100mM MgCl$_2$

250mM NaCl.

T7: 3 units of modified T7 DNA Polymerase

LM: 1µl DTT (0.1M)

2µl 1 x labelling mix

tA: 2.5µl of ddATP termination mix

tC: 2.5µl of ddCTP termination mix

tG: 2.5µl of ddGTP termination mix

tT: 2.5µl of ddTTP termination mix

The wells were dried down, sealed and stored at -20°C.

## The Sequencing Reaction

1. 25µl of DNA template to be sequenced (in water or TE buffer) was added to well P. The cover seal was replaced.

2. The template primer mix was incubated at 65°C for 2 minutes, and then allowed to cool to room temperature.

3. The mixture was then transferred to well T7.

4. 1µl radioactive label ([α $^{32}$P]-dATP, [$^{35}$S-dATPαS]) was added to well T7.

5. The contents of well T7 were transferred to LM well and incubated at room temperature for 5 minutes

6. 6µl sub aliquots of the mix now in LM well were transferred into consecutive tA tC tG tT wells and incubated at 37°C for 5 minutes.

7. 4µl of loading buffer was added to each termination well.

8. Wells were placed at 90°C for 3 minutes, and then the fluid reaction products loaded onto a sequencing gel.

## Results

1. Autoradiogram of $^{35}$S labelled DNA after electrophoresis.

The results showed:

1. Good resolution of DNA.

2. Sequencing ladder can be read easily, for at least 200 nucleotides.

3. Little background and no compression or stuttering of the enzyme.

2. Essentially similar results were obtained when using a $^{32}$P radiolabel.

## Example 6

### DNA ligation

Wells were washed in ethanol, washed 3 times in double-distilled water and dried.

The following solutions were used and mixed as described.

T4 DNA ligase (6/19): 1.5U(Weiss)/µl in 25mM Tris/HCl, pH7.5, 1mM DTT.

Ligation buffer (10x): 0.5M Tris/HCl, pH7.4, 10mM MgCl$_{12}$, 100mM DTT, 10mm ATP

T4 DNA ligase solution was prepared:

40µl T4 DNA ligase

40µl ligation buffer (10x)

80µl double-distilled water.

4μl this solution was added to each well and lyophilised under vacuum until dry. This process was complete in 75 minutes. Dried wells were sealed and stored at -20°C.

Assaying for ligase activity.

Solutions used.

EcoR V digested PBR322 DNA (10μg/μl), dephosphorylated with calf intestinal alkaline phosphatase.
Hae 111 digested DNA (10μg/100μl).
Assay mixture
2μl EcoR V/PBR322 DNA
2μl Hae 111/DNA
16μl ddH$_2$O

1. 1.6μl DNA assay mixture was added to each well with 8.4μl double-distilled water.
2. Enzyme was gently resuspended using a pipette, the wells sealed and incubated at 37°C for 60 min.
3. 20μl double-distilled water was added to each well and mixed on ice.
4. 5μl each ligation was added to 80μl competent E.Coli NM554 in individual tubes.
5. Cells were left on ice for 30 minutes.
6. Cells were heated in a 42°C water bath for 45 seconds and placed on ice for 3 minutes.
7. 900μl L broth (10g/l NaCl; 10g/l Bactotryptone, 5g/l Bactoyeast extract) was added to each tube.
8. Tubes were shaken at 37°C for 60 minutes.
9. 10μl each sample was plated onto L agar (L broth with 15g/l Bactoagar), containing 25μg/ml ampicillin, and incubated overnight at 37°C.
10. Colonies were counted and used as the unit of assay.

Results

T4 DNA ligase dried in wells in the presence of buffers showed a 65% retention of activity when compared, in this assay, with identical samples of ligase prepared simultanlously but stored on ice and not lyophilised.

## Claims

1. A method of performing a nucleic acid reaction, by providing a reaction vessel containing a predetermined amount of a reagent for the reaction, comprising a mixture of an enzyme capable of acting on nucleic acids and a buffer and including components of the reaction except a nucleic acid substrate, in a dried state, adding to the vessel aqueous fluid containing a nucleic acid sequence, and incubating the vessel.

2. A method of performing a nucleic acid manipulation, by providing a first and at least one subsequent vessel, each vessel containing a predetermined amount of a reagent for a nucleic acid reaction in a dried state, adding to the first vessel aqueous fluid containing a nucleic acid sequence, and transferring a fluid reaction product from the first vessel to the or each subsequent vessel.

3. A method as claimed in Claim 2, wherein the nucleic acid manipulation is a sequencing operation.

4. A method as claimed in Claim 3. wherein the sequencing is a dideoxy sequencing operation.

5. A method as claimed in any one of Claims 2 to 4, wherein the reagent is an enzyme capable of acting on nucleic acids or a substrate for such enzyme.

6. A method as claimed in any one of Claims 2 to 5, wherein the or each vessel contains a mixture of an enzyme and a buffer in a dried state.

7. A method as claimed in any one of Claims 2 to 6, wherein at least one vessel contains a predetermined amount of a radioactively labelled reagent in a dried state.

8. A method as claimed in any one of Claims 1 to 7, wherein the reagent is an enzyme selected from nucleic acid polymerases and nucleic acid modification enzymes.

9. A method as claimed in Claim 1, wherein the reagent is a restriction enzyme.

10. A method as claimed in Claim 1 or Claim 6, wherein the buffer is a reaction buffer.

## Patentansprüche

1. Verfahren zur Durchführung einer Nukleinsäure-Reaktion, in dem ein Reaktionsgefäß zur Verfügung gestellt wird, das eine vorbestimmte Menge eines Reagenzes für die Reaktion, umfassend eine Mischung eines Enzyms, das auf Nukleinsäuren wirken kann und einen Puffer, enthält und die Reaktions-Komponenten mit Ausnahme eines Nukleinsäure-Substrats einschließt in einem getrockneten Zustand, zu dem Gefäß wäßrige Flüssigkeit, die eine Nukleinsäure-Sequenz enthält, zugibt und das Gefäß inkubiert.

2. Verfahren zur Durchführung einer Nukleinsäure-Manipulation, in dem ein erstes und mindestens ein nachfolgendes Gefäß zur Verfügung gestellt wird, wobei jedes Gefäß eine vorbestimmte Menge eines Reagenzes für eine Nukleinsäure-Reaktion in einem getrockneten Zustand enthält, zu dem ersten Gefäß eine wäßrige Flüssigkeit, die eine Nukleinsäure-Sequenz enthält, zugegeben wird und ein flüssiges Reaktionsprodukt von dem ersten Gefäß in das oder jedes nachfolgende Gefäß transferiert wird.

3. Verfahren nach Anspruch 2, worin die Nukleinsäure-Manipulation ein Sequenzierungs-Verfahren ist.

4. Verfahren nach Anspruch 3, worin die Sequenzierung ein Dideoxy-Sequenzierungs-Verfahren ist.

5. Verfahren nach einem der Ansprüche 2 bis 4, worin das Reagenz ein Enzym ist, das auf Nukleinsäuren wirken kann oder ein Substrat für ein solches Enzym ist.

6. Verfahren nach einem der Ansprüche 2 bis 5, worin das oder jedes Gefäß eine Mischung eines Enzyms und eines Puffers in einem getrockneten Zustand enthält.

7. Verfahren nach einem der Ansprüche 2 bis 6, worin mindestens ein Gefäß eine vorbestimmte Menge eines radioaktiv markierten Reagenzes in getrocknetem Zustand enthält.

8. Verfahren nach einem der Ansprüche 1 bis 7, worin das Reagenz ein Enzym ist, ausgewählt aus Nukleinsäure-Polymerasen und Nukleinsäure-Modifizierungs-Enzymen.

9. Verfahren nach Anspruch 1, worin das Reagenz ein Restriktionsenzym ist.

10. Verfahren nach Anspruch 1 oder Anspruch 6, worin der Puffer ein Reaktionspuffer ist.

**Revendications**

1. Méthode pour exécuter une réaction sur un acide nucléique, consistant à fournir un récipient réactionnel contenant une quantité prédéterminée d'un réactif pour la réaction, à l'état séché, comprenant un mélange d'une enzyme capable d'agir sur des acides nucléiques et un tampon, et incluant les composants de la réaction, à l'exception d'un substrat consistant en acide nucléique, à ajouter au récipient un liquide aqueux contenant une séquence d'acide nucléique, et à mettre le récipient en incubation.

2. Méthode pour exécuter une manipulation d'acide nucléique, consistant à fournir un premier récipient et au moins un récipient suivant, chaque récipient contenant une quantité prédéterminée d'un réactif, à l'état séché, pour une réaction sur un acide nucléique, à ajouter au premier récipient un liquide aqueux contenant une séquence d'acide nucléique, et à transférer le produit réactionnel liquide du premier récipient au récipient suivant ou à chacun des récipients suivants.

3. Méthode selon la revendication 2, dans laquelle la manipulation d'acide nucléique est une opération de séquençage.

4. Méthode selon la revendication 3, dans laquelle le séquençage est une opération de séquençage par didésoxynucléotides.

5. Méthode selon l'une quelconque des revendications 2 à 4, dans laquelle le réactif est une enzyme capable d'agir sur des acides nucléiques, ou un substrat pour une telle enzyme.

6. Méthode selon l'une quelconque des revendications 2 à 5, dans laquelle le ou chaque récipient contient un mélange d'une enzyme et d'un tampon à l'état séché.

7. Méthode selon l'une quelconque des revendications 2 à 6, dans laquelle au moins un récipient contient une quantité prédéterminée d'un réactif radiomarqué à l'état séché.

8. Méthode selon l'une quelconque des revendications 1 à 7, dans laquelle le réactif est une enzyme choisie parmi les (acide nucléique)-polymérases et les enzymes de modification d'acides nucléiques.

9. Méthode selon la revendication 1, dans laquelle le réactif est une enzyme de restriction.

10. Méthode selon la revendication 1 ou la revendication 6, dans laquelle le tampon est un tampon de réaction.